# EUROPÄISCHE PATENTSCHRIFT

(11) **EP 1 687 251 B1**
(45) Veröffentlichungstag und Bekanntmachung des Hinweises auf die Patenterteilung: **18.03.2009**
(21) Anmeldenummer: 04797820.0
(22) Anmeldetag: 11.11.2004
(51) Int. Cl.: C07C 67/08, C07C 67/14, C07C 67/02, C07C 69/54

(54) **VERFAHREN ZUR VERESTERUNG VON ALKOHOLEN MIT OLEFINISCH UNGESÄTTIGTEN CARBONSÄUREN**
METHOD FOR THE ESTERIFICATION OF ALCOHOLS WITH OLEFINICALLY UNSATURATED CARBOXYLIC ACIDS
PROCEDE D'ESTERIFICATION D'ALCOOLS AU MOYEN D'ACIDES CARBOXYLIQUES A INSATURATION OLEFINIQUE

(30) Priorität: 22.11.2003 DE 10354652
(43) Veröffentlichungstag der Anmeldung: 09.08.2006
(73) Patentinhaber: Clariant Produkte (Deutschland) GmbH, 65929 Frankfurt am Main (DE)
(72) Erfinder: GLOS, Martin, 45145 Essen (DE)
(74) Vertreter: Mikulecky, Klaus
(86) Internationale Anmeldenummer: PCT/EP2004/012790
(87) Internationale Veröffentlichungsnummer: WO 2005/049544

(56) Entgegenhaltungen:
- JP-A- 2003 313 153
- US-A1- 2001 023 302
- US-A1- 2002 193 624

## Beschreibung

Die vorliegende Erfindung betrifft ein Verfahren zur Herstellung von Estern mit olefinisch ungesättigten Carbonsäuren, bei dem man Oxazoline (4,5-Dihydro-1,3-oxazole) als Stabilisatoren verwendet.

Unter den Estern der olefinisch ungesättigten Carbonsäuren sind besonders die (Meth)acrylsäureester technisch interessant, da sie Ausgangsverbindungen zur Herstellung von Polymeren und Copolymere darstellen, die in vielfältigen Bereichen Anwendungen finden. Der Begriff (Meth)acrylsäureester steht für Methacrylsäureester und Acrylsäureester. Die vorliegende Erfindung ist mit besonderem Vorteil zur Herstellung dieser Ester anwendbar und wird nachfolgend am Beispiel von (Meth)acrylsäureestern beschrieben.

Bei der Herstellung der Ester von Alkoholen mit olefinisch ungesättigten Carbonsäuren sind verschiedene Probleme zu beachten. Zum einen neigen olefinisch ungesättigte Carbonsäuren unter dem Einfluss von Hitze oder Licht zur Polymerisation. Vor allem bei der Herstellung und destillativen Reinigung sind sie Temperaturen ausgesetzt, die leicht eine unerwünschte Polymerisation auslösen können. Die Polymerbildung kann auch dadurch verursacht werden, dass am kälteren Reaktordeckel oder anderen Bauteilen der Anlage die Dämpfe der nicht stabilisierten ungesättigten Carbonsäure kondensieren und dort polymerisieren. Die Folge ist eine Verschmutzung der Apparaturen, ein Verstopfen von Leitungen und Pumpen und die Belegung von Kolonnenböden und Wärmetauscheroberflächen ("fouling"). Das Reinigen der Anlagen ist ein aufwändiger, teurer und umweltbelastender Vorgang, der zudem die Verfügbarkeit der Anlagen stark reduziert.

Der Stand der Technik offenbart verschiedene Verfahren, um den Reinigungsaufwand gering zu halten.

US-2001/023 302 offenbart ein Verfahren zum Herstellen eines optisch aktiven Hemiesters der Formel (1) 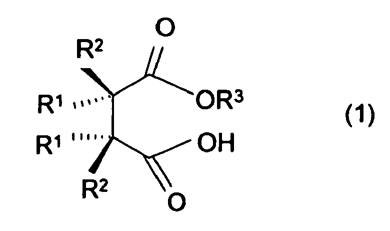 worin R¹, R² und R³ die gleichen Bedeutungen darstellen, wie unten beschrieben, welches die Umsetzung eines zyklischen Säureanhydrids der Formel (2): 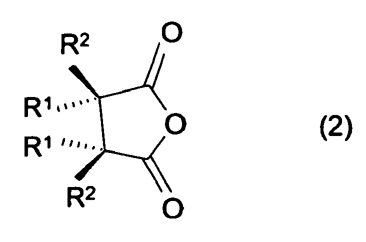 worin R¹ und R² verschieden sind und unabhängig voneinander ein Wasserstoffatom, ein Halogenatom, eine Alkylgruppe, welche wahlweise mit einer Alkoxylgruppe oder einem Halogenatom und ähnlichem substituiert ist, darstellen, mit einer Hydroxyverbindung der Formel (3):

R³OH (3)

worin R³ eine Alkylgruppe, welche wahlweise mit einer Alkoxylgruppe, einer Phenoxygruppe, einer Dialkylaminogruppe oder einem Halogenatom und ähnlichem substituiert ist, darstellt, in Anwesenheit eines asymmetrischen Katalysators umfasst.

US-2002/193 624 offenbart ein Verfahren zur Herstellung von Alkylpolyalkylenglykolcarbonsäureestern umfassend die Umsetzung eines Carbonsäureesters mit einem Alkylenoxid, wobei für die Umsetzung eine Multimetallcyanid-Verbindung der allgemeinen Formel (1)

M³_{z}M¹ₐ[M²(CN)_{b}(A)_{c}]_{d} · fM¹_{g}Xₙ · mM³ₚY_{q} · h(H₂O) · eL · kP (1)

als Katalysator eingesetzt wird, sowie die Verwendung der erfindungsgemäß hergestellten Alkylpolyalkylenglykolcarbonsäureestern als Rohstoffe für Betonverflüssigerpolymere.

JP-2003 313 153 offenbart ein Verfahren zur Herstellung optisch aktiver 1,2-Diole durch Reaktion von Carbonsäurehalogeniden in Gegenwart optisch aktiver Oxazolin-Kupfer-Komplexe und einer Base.

DE 100 36 879 A1 beschreibt ein Verfahren zur Herstellung von Estern der (Meth)acrylsäure durch Umesterung, bei dem durch Rückführung der organischen Phase des Destillats aus dem nicht trennwirksamen Bereich der Kolonne in die Reaktionszone die Polymerablagerung in den Kolonnen verhindert wird.

DE 101 27 938 A1 beschreibt ein Verfahren zur Herstellung von (Meth)acrylsäureestern durch Umesterung, wobei ein inertes Gas oder Gasgemisch durch die Reaktionszone geleitet wird. Zusätzlich wird eine Stabilisatorlösung in die Kolonnen eingesprüht. Dadurch wird verhindert, dass sich Polymerisat auf den Wärmetauscheroberflächen bildet.

DE 100 63 175 A1 beschreibt die Direktveresterung von höheren Alkoholen. Das Verfahren beruht darauf, dass in einem Reaktor mit Umlaufverdampfer gearbeitet wird und ein Teil des Destillats (Cyclohexan als Schleppmittel) in dem Umlaufverdampfer zurückgeführt wird. Dadurch kann man auf Kupfersalze als Stabilisatoren verzichten.

DE 100 63 176 A1 beschreibt ein Verfahren zur Herstellung von höheren (Meth)acrylsäureestern durch Direktveresterung von höheren Alkoholen. Es wird Cyclohexan als Schleppmittel verwendet. Das Verfahren beruht darauf, dass ein Teil der Packung in der Destillationskolonne aus Kupfer oder einem kupferhaltigen Werkstoff besteht. Dadurch kann man auf Kupfersalze als Stabilisatoren verzichten.

DE 100 63 511 A1 beschreibt ein Verfahren zur Herstellung von Alkylpolyalkylenglykolestern monoethylenisch ungesättigter Carbonsäuren über Direktveresterung. Es wird bei Normaldruck oder Überdruck verestert und dann nur kurzzeitig bei reduziertem Druck das Wasser abdestilliert und anschließend wieder bei Normaldruck oder erhöhtem Druck die Veresterung fortgesetzt.

EP 874 870 A1 beschreibt ein Verfahren zur Herstellung von Polyglykol(meth)acrylaten durch Umesterung. Als Katalysatoren werden Ca(OH)₂ oder Mischungen mit LiCl eingesetzt. Als Stabilisatoren dienen Hydrochinone oder Phenole oder sterisch gehinderte Amine.

Aufgabe vorliegender Erfindung war es, verbesserte Stabilisatoren für olefinisch ungesättigte Carbonsäuren aufzufinden.

Überraschenderweise wurde nun gefunden, dass Ester von olefinisch ungesättigten Carbonsäure unter Verwendung von Oxazolinen hergestellt werden können. Durch den Einsatz der Oxazoline wird verhindert, dass die verwendeten Apparaturen wie z.B. Leitungen, Kolonnen und Wärmetauscher durch Ablagerungen von Polymerisaten verstopft oder verschmutzt werden. Dadurch sinkt der Aufwand zur Reinigung der Anlagen und die Verfügbarkeit der Anlage wird verbessert.

Gegenstand der Erfindung ist somit ein Verfahren zur Herstellung von Estern aus Alkoholen und olefinisch ungesättigten Carbonsäuren durch Umsetzung eines Alkohols mit einer olefinisch ungesättigten Carbonsäure oder einem reaktiven Derivat einer solchen, wobei 1 ppm bis 1 Gew.-%, bezogen auf das Gewicht des Reaktionsgemischs aus Alkohol und olefinisch ungesättigter Carbonsäure/Carbonsäurederivat, mindestens eines Oxazolins der Formel 1 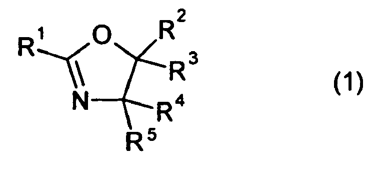 zugegen ist, worin R¹, R², R³, R⁴ und R⁵ unabhängig voneinander für Wasserstoff oder Kohlenwasserstoffreste mit 1 bis 12 Kohlenstoffatomen stehen, und R¹, R², R³, R⁴ und R⁵ gleich oder verschieden sein können.

Ein weiterer Gegenstand der Erfindung ist die Verwendung von Verbindungen der Formel 1 als Stabilisatoren in der Umsetzung zwischen Alkoholen und olefinisch ungesättigten Carbonsäuren oder deren reaktiven Derivaten, wobei 1 ppm bis 1 Gew.-%, bezogen auf das Gewicht des Reaktionsgemischs aus Alkohol und Carbonsäure/Carbonsäurederivat, der Verbindung der Formel 1 zur Anwendung kommen.

Ein weiterer Gegenstand der Erfindung ist eine Zusammensetzung, enthaltend
A) einen Alkohol
B) eine olefinisch ungesättigte Carbonsäure oder ein reaktives Derivat einer solchen,
   wobei das molare Verhältnis zwischen A) : B) zwischen 1 : 0,2 und 1 : 15 liegt, sowie
C) 1 ppm bei 1 Gew.-%, bezogen auf das Gesamtgewicht von A) und B), einer Verbindung der Formel 1 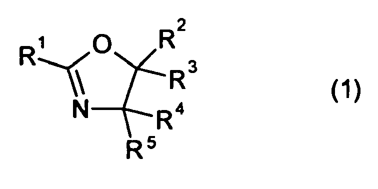
worin R¹, R², R³, R⁴ und R⁵ Wasserstoff, verzweigte, unverzweigte, cyclische, gesättigte oder ungesättigte Kohlenwasserstoffreste mit bis zu 12 C-Atomen, die durch Heteroatome substituiert sein können, bedeuten, und R¹, R², R³, R⁴ und R⁵ gleich oder verschieden sein können.

In einer bevorzugten Ausführungsform stehen R¹, R², R³, R⁴ und R⁵ unabhängig voneinander für Wasserstoff oder Methylgruppen.

In einer besonders bevorzugten Ausführungsform der Erfindung bedeuten
- R¹: Methyl
- R² und R³: Wasserstoff
- R⁴ und R⁵: Wasserstoff oder Methyl.

Die erfindungsgemäßen Oxazoline werden in Mengen von bevorzugt 10 ppm bis 0,5 Gew.-%, besonders 50 ppm bis 0,1 Gew.-% bezogen auf das Reaktionsgemisch aus Alkohol und Carbonsäure/Carbonsäurederivat eingesetzt.

Zur Herstellung der erfindungsgemäßen Ester können verschiedene Methoden verwendet werden wie z.B. die Umsetzung von reaktiven Carbonsäurederivaten, wie beispielsweise Säurehalogenide oder Ester, mit den Alkoholen, oder die Direktveresterung der olefinisch ungesättigte Carbonsäure mit Alkoholen. Es kann sowohl mit als auch ohne Lösungsmittel gearbeitet werden.

Das erfindungsgemäße Verfahren eignet sich generell für die Herstellung von Estern ungesättigter Carbonsäuren, bevorzugt alpha, beta-ungesättigter Carbonsäureester und besonders bevorzugt für Ester olefinisch ungesättigter Monocarbonsäuren mit 3 bis 6 Kohlenstoffatomen und olefinisch ungesättigter Dicarbonsäuren mit 4 bis 8 Kohlenstoffatomen.

Als monoethylenisch ungesättigte Mono- und Dicarbonsäuren kommen bevorzugt Acrylsäure, Methacrylsäure, Styrolsulfonsäure, Maleinsäure, Fumarsäure, Crotonsäure, Itaconsäure und Seneciosäure in Betracht. Es können auch die entsprechenden Anhydride wie z.B. Maleinsäureanhydrid, oder die entsprechenden Säurechloride verwendet werden. Bei der Umesterung kommen die entsprechenden Säureester wie z.B. die Methyl- oder Ethylester als Ausgangsmaterial zum Einsatz.

Als Alkohole können ein- oder mehrwertige Alkohole, die verzweigt, unverzweigt oder cyclisch, gesättigt oder ungesättigt sein können, mit bis zu 500 Kohlenstoffen, eingesetzt werden. Solche Alkohole sind beispielsweise
C₁ bis C₃₀-Monoalkohole, wie beispielsweise 2-Ethylhexyl-, 2-Propylheptyl-, Lauryl-, Stearyl- oder Behenylalkohol;
C₁ bis C₁₂-Alkyl-substituierte Cyclopentanole oder Cyclohexanole, wie beispielsweise tert.-Butylcyclohexanol;
C₂ bis C₂₀-Diole, wie beispielsweise Ethylenglykol, Phenylethylenglykol, 1,2- und 1,3-Propylenglykol, 1,2-, 1,3-,oder 1,4-Butylenglykol, 1,6-Hexandiol, und deren Mono-C₁- bis C₃₀-alkylether;
aromatische ein- oder mehrwertige Alkohole wie beispielsweise Phenole, Resorcin oder Tannin,
Polyethylen- und Polypropylenglykole, wie beispielsweise Diethylenglykol, Triethylenglykol, Tetraethylenglykol, Dipropylenglykol, Tripropylenglykol und deren Mono-C₁- bis C₃₀-alkylether;
Triole und höhere Polyole, wie beispielsweise Glycerin, Polyglycerine, Trimethylolpropan, Pentaerythrit oder C₁- bis C₃₀-alkylether davon mit mindestens einer freien Hydroxylgruppe;
die alkoxylierten Derivate der genannten Alkohole, wie beispielsweise Ethoxylate, Propoxylate, Butoxylate.

Zur Herstellung der alkoxylierten Alkohole oder Phenole wird ein Mol eines Alkohols oder Phenols mit 1 bis 300 Mol eines Alkylenoxids mit 2 bis 20 C-Atomen umgesetzt. Als Alkylenoxide kommen beispielsweise Ethylenoxid, Propylenoxid, Butylenoxid oder Styroloxid in Betracht. Man kann sowohl ein Alkylenoxid oder mehrere Alkylenoxide and den Alkohol oder das Phenol anlagern. Werden mehrere Alkylenoxide angelagert so können die einzelnen Alkylenoxide im Produkt statistisch verteilt oder in Blöcken angeordnet sein.

Beispiele für alkoxylierte Alkohole sind Methylpolyethylenglykole die 3, 10, 25, 40, 100 oder 200 Mol Ethylenoxid enthalten, die durch Umsetzung von Methanol mit 3, 10, 25, 40, 100 oder 200 Mol Ethylenoxid erhalten werden. Entsprechend können auch langkettige Alkohole wie z.B. Butanol, Dodecylalkohol, Isododecylalkohol, Strearylalkohol, Behenylalkohol, Oleylalkohol, C₈- bis C₂₀-Oxoalkohole, C₃- bis C₂₀-Alkylphenole oder Arylphenole mit entsprechenden Mengen Ethylenoxid umgesetzt werden.

Ebenso können Alkohole die weitere Heteroatome enthalten eingesetzt werden, wie beispielsweise Alkoxylate von primären oder sekundären Aminen, wie beispielsweise Oleylaminethoxylate, Didecylaminethoxylate oder Kokosfettaminpropoxylate und Amidalkoxylate, wie beispielsweise Ölsäureamidethoxylate.

Auch sind Verbindungen wie Ethanolamin, Diethanolamin, Triethanolamin, 2-Dimethylethan-1-ol, 3-Dimethylaminopropan-1-ol, 1-Dimethylaminopropan-2-ol, 2-Dimethylaminopropan-1-ol, 6-Dimethylaminohexan-1-ol, 2-Diethylaminoethyn-1-ol, 3-Diethylaminopropan-1-ol, 6-Diethylaminohexan-1-ol, 2-Dibutylaminoethan-1-ol, 3-Dibutylaminopropan-1-ol, und 6-Dibutylaminohexan-1-ol als Alkohole im Sinne vorliegender Erfindung einsetzbar.

Das molare Verhältnis Alkohol : ungesättigte Carbonsäure liegt vorzugsweise im Bereich 1: 0,2 bis 15, insbesondere im Bereich 1:0,8 bis 15.

Neben dem erfindungsgemäßen Stabilisator nach Formel 1 können die nach dem Stand der Technik bekannten Stabilisatoren verwendet werden. Übliche Stabilisatoren sind N-Oxyle wie z.B. 4-Hydroxy-2,2,6,6-tetramethyl-1-oxyl-piperidin , 4-Oxo-2,2,6,6-tetramethylpiperidn-N-Oxy, Phenole und Naphthole wie z.B. Hydrochinon, Naphthochinon, p-Aminophenol, p-Nitrosophenol, 2-tert.-Butylphenol, 4-tert.-Butylphenol, 2,4-Di-tert.-Butylphenol, 2-Methyl-4-tert.-Buthylphenol, 4-Methyl-2,3-di-tert.-butylphenol, lonol K 65^{®}, p-Methoxyphenol, Butylhydroxyanisol oder 4-Amine, wie z.B. N,N-Diphenylamin, Phenylendiamine, wie z.B. N,N'-Dialkyl-para-phenylendiamene, wobei die Alkylreste gleich oder verschieden sein können, Hydroxylamine, wie z.B. N,N- Diethylhydroxylamin, phosphorhaltige Verbindungen, wie z.B. Triphenylphosphin, Triphenylphosphit oder Triethylphosphit oder schwefelhaltige Verbindungen, wie z.B. Schwefeldioxid, Diphenylsulfid, Phenothiazin oder 5-tert.-Butyl-4-hydroxy-2-methylphenylsulfid sowie Irganox^{©}-Typen, Cupferron-Typen^{©} und Kupfer-Salze.

Diese Verbindungen können einzeln oder in Mischungen eingesetzt werden. Die Mengen reichen von 10 ppm bis 5 Gew.-% meistens von 50 ppm bis 3 Gew.-% bezogen auf den eingesetzten Alkohol. Die Reaktion kann in einer Inertgasatmosphäre (wie z.B. Stickstoff, Argon, Helium) oder auch gegebenenfalls unter Zugabe von Luft oder sauerstoffhaltigen Gasgemischen durchgeführt werden.

Bei der Veresterung können die nach dem Stand der Technik bekannten Katalysatoren verwendet werden. Übliche Veresterungskatalysatoren sind Schwefelsäure, schweflige Säure, Dischwefelsäure, Polyschwefelsäuren, Schwefeltrioxid, Methansulfonsäure, Benzolsulfonsäure, C₁-C₃₀-Alkylbenzolsulfonsäure, Naphthalinsulfonsäure, Schwefelsäuremonoester von C₁-C₃₀-Alkoholen wie Dodecylsulfat, Phosphorsäure, phosphorige Säure, unterphosphorige Säure, Polyphosphorsäure, Phosphorsäureester von C₁-C₃₀-Alkoholen, Chlorwasserstoffsäure, Perchlorsäure, saure Ionentauscher, Heteropolysäuren, "solid super acids", sowie Salze dieser Säuren, Lewissäuren wie Bortrichlorid, Aluminiumsulfat und Eisentrichlorid.

Bei der Umesterung können sämtliche im Stand der Technik beschriebenen Katalysatoren verwendet werden wie beispielsweise Magnesium-, Aluminium- oder Titan-Alkoholate wie Tetramethyl-, Tetraethyl, Tetraisopropyl-, Tetrapropyl, Tetraisobutyl, und Tetrabutyltitanat, Titanphenolate, Zirkoniumalkoholate oder auch Alkoholate mit weiteren Funktionalitäten in den Liganden, Metallchelatverbindungen von beispielsweise Hafnium, Titan, Zirkon oder Calcium, Alkali- und Magnesiumalkoholate, organische Zinnverbindungen wie Dibutylzinnoxid oder Dibutylzinnoxychlorid oder Calcium- und Lithiumverbindungen, beispielsweise -Oxide, -Hydroxide, -Carbonate oder -Halogenide, Cyanate von Alkalimetallen oder Erdalkalimetallen oder basische Trägerkatalysatoren.

Der Katalysator wird gegebenenfalls in Mengen von vorzugsweise 0,01 bis 10, insbesondere 0,05 bis 5 Gew.-% bezogen auf das gesamte Reaktionsgemisch eingesetzt.

Die Veresterungen können bei Temperaturen von 40 bis 180°C durchgeführt werden. Bevorzugt wird im Bereich von 80 bis 140°C gearbeitet. Die Veresterung wird vorzugsweise mit einem Überschuss an ungesättigter Carbonsäure durchgeführt. Vorzugsweise wird in einem Druckbereich von 1 mbar bis 10 bar gearbeitet.

Die Veresterung kann kontinuierlich oder diskontinuierlich durchgeführt werden.

### Beispiele

### Beispiel 1

In einem emaillierten Reaktor mit einem Volumen von 2,2 m³ wurden 1400 kg eines Methylpolyethylenglykols der Molmasse 1100, 175 kg Methacrylsäure, 9,7 kg konz. (97 %) Schwefelsäure, 510 g Phenothiazin und 158 g 2-Methyloxazolin vorgelegt. Es wurde unter Durchleiten von Stickstoff für 8 Stunden auf 125°C erwärmt. Anschließend wurde der Druck innerhalb von 8 Stunden langsam auf 50 mbar abgesenkt und dort für 14 Stunden gehalten. Hierbei wurde als Destillat ein Gemisch aus Methacrylsäure und Wasser abgenommen. Über NMR wurde ein Umsetzungsgrad von 97 % ermittelt. Nach dem Entleeren konnten im Kessel und in den Anlagenteilen zur Destillation keine Ablagerungen oder Verschmutzungen durch Polymerisate festgestellt werden.

### Beispiel 2

In einem emaillierten Reaktor mit einem Volumen von 2,2 m³ wurden 1166 kg eines mit Laurylalkohol gestarteten Ethoxylats mit durchschnittlich 7 Einheiten Ethylenoxid, 319 kg Methacrylsäure, 11,7 kg konz. (97%) Schwefelsäure, 3,2 kg Unterphosphorige Säure, 920 g Phenothiazin, 280 g para-Methoxyphenol und 233 g 2-Methyloxazolin vorgelegt. Es wurde unter Durchleiten von Stickstoff für 3 Stunden auf 125°C erwärmt. Anschließend wurde der Druck innerhalb von 8 Stunden langsam auf 50 mbar abgesenkt und dort für 3 Stunden gehalten. Hierbei wurde als Destillat ein Gemisch aus Methacrylsäure und Wasser abgenommen. Über NMR wurde ein Umsetzungsgrad von 99 % ermittelt. Nach dem Entleeren konnten im Kessel und in den Anlagenteilen zur Destillation keine Ablagerungen oder Verschmutzungen durch Polymerisate festgestellt werden.

### Beispiel 3

In einem emaillierten Reaktor mit einem Volumen von 2,2 m³ wurden 990 kg Behenylalkohol, 543 kg Xylol, 266 kg Acrylsäure, 16,5 kg para-Toluolsulfonsäure, 2,3 kg para-Methoxyphenol und 247 g 2-Methyloxazolin vorgelegt. Nun wurde auf 130°C erwärmt und innerhalb von 6 Stunden die Temperatur auf 170°C erhöht. Dabei wurde das anfallende Reaktionswasser als Azeotrop abdestilliert und das Xylol wieder in die Reaktion zurückgeführt. Nachdem keine Wasserabscheidung mehr festgestellt werden konnte wurde über NMR ein Umsetzungsgrad von 98 % ermittelt. Nach dem Entleeren konnten im Kessel, der Kolonne und dem Abscheider keine Ablagerungen oder Verschmutzungen durch Polymerisate festgestellt werden.

### Beispiel 4

In einem emaillierten Reaktor mit einem Volumen von 2,2 m³ wurden 1300 kg eines Polyethylenglykols mit einer mittleren Molmasse von 600, 259 kg Methacrylsäure, 11,5 kg konz. (97 %) Schwefelsäure, 1044 g Phenothiazin, 396 g lonol K 65^{©} und 466 g 2-Methyloxazolin vorgelegt. Es wurde unter Durchleiten von Stickstoff für 4 Stunden bei 500 mbar auf 125°C erwärmt. Anschließend wurde der Druck innerhalb von 4 Stunden langsam auf 50 mbar abgesenkt und dort für 4 Stunden gehalten. Hierbei wurde als Destillat ein Gemisch aus Methacrylsäure und Wasser abgenommen. Über NMR wurde ein Umsetzungsgrad von 98 % ermittelt. Nach dem Entleeren konnten im Kessels und in den Anlagenteilen zur Destillation keine Ablagerungen oder Verschmutzungen durch Polymerisate festgestellt werden.

### Beispiel 5

In einem emaillierten Reaktor mit einem Volumen von 2,2 m³ wurden 1400 kg eines Methylpolyethylenglykols mit einer mittleren Molmasse von 750, 259 kg Methacrylsäure, 9,5 kg konz. (97 %) Schwefelsäure, 746 g Phenothiazin, 280 g lonol K 65^{©} und 233 g 2-Methyloxazolin vorgelegt. Es wurde unter Durchleiten von Stickstoff für 2 Stunden bei 500 mbar auf 125°C erwärmt. Anschließend wurde der Druck innerhalb von 10 Stunden langsam auf 50 mbar abgesenkt und dort für 4 Stunden gehalten. Hierbei wurde als Destillat ein Gemisch aus Methacrylsäure und Wasser abgenommen. Über NMR wurde ein Umsetzungsgrad von 96 % ermittelt. Nach dem Entleeren konnten im Kessel und in den Anlagenteilen zur Destillation keine Ablagerungen oder Verschmutzungen durch Polymerisate festgestellt werden.

### Beispiel 6 (Vergleichsbeispiel ohne Oxazolin)

In einem emaillierten Reaktor mit einem Volumen von 2,2 m³ wurden 1400 kg eines Methylpolyethylenglykols mit einer mittleren Molmasse von 750, 259 kg Methacrylsäure, 9,5 kg konz. (97 %) Schwefelsäure, 746 g Phenothiazin und 280 g Ionol K 65^{®} vorgelegt. Es wurde unter Durchleiten von Stickstoff für 2 Stunden bei 500 mbar auf 125°C erwärmt. Anschließend wurde der Druck innerhalb von 10 Stunden langsam auf 50 mbar abgesenkt und dort für 4 Stunden gehalten. Hierbei wurde als Destillat ein Gemisch aus Methacrylsäure und Wasser abgenommen. Über NMR wurde ein Umsetzungsgrad von 96% ermittelt. Nach dem Entleeren waren im Kessel Ablagerungen und Verschmutzungen durch Polymerisate vorzufinden. Ebenso hatten sich in den Anlagenbereichen, in denen das Destillat kondensierte, Polymerisate gebildet.

### Beispiel 7

In einem Reaktor mit einem Volumen von 2,1 m³ wurden 1189 kg eines mit Laurylalkohol gestarteten Ethoxylats mit durchschnittlich 7 Einheiten Ethylenoxid, 497 kg Methacrylsäuremethylester, 25,9 kg Titantetraisopropylat, 770 g Butylhydroxytoluol, 442 g para-Methoxyphenol und 178 g 2-Methyloxazolin vorgelegt. Es wurde unter Durchleiten von Stickstoff auf 105°C erwärmt und begonnen, das gebildete Methanol zusammen mit überschüssigem Methacrylsäuremethylester abzudestillieren. Anschließend wurde die Temperatur innerhalb von 7 Stunden langsam auf 150°C erhöht und dort für 2 Stunden gehalten. Schließlich wurde die Temperatur auf 130°C gesenkt und bei 5 mbar Druck der überschüssige Methacrylsäuremethylester abdestilliert. Über NMR wurde ein Umsetzungsgrad von 99 % ermittelt. Nach dem Entleeren konnten im Kessel und in den Anlagenteilen zur Destillation keine Ablagerungen oder Verschmutzungen durch Polymerisate festgestellt werden.

### Beispiel 8

In einem Reaktor mit einem Volumen von 2,1 m³ wurden 1109 kg N,N-Didecylaminoethanol, 586 kg Methacrylsäuremethylester, 3,2 kg Lithiumhydroxid, 1,9 kg Phenothiazin und 320 g 2-Methyloxazolin vorgelegt. Es wurde unter Durchleiten von Stickstoff für 8 Stunden auf 105°C erwärmt und das gebildete Methanol zusammen mit überschüssigem Methacrylsäuremethylester abdestilliert. Anschließend wurde der Druck innerhalb von 6 Stunden langsam auf 300 mbar abgesenkt und dort für 2 Stunden gehalten. Über NMR wurde ein Umsetzungsgrad von 96 % ermittelt. Nach dem Entleeren konnten im Kessel und in den Anlagenteilen zur Destillation keine Ablagerungen oder Verschmutzungen durch Polymerisate festgestellt werden.

### Beispiel 9

In einem Reaktor mit einem Volumen von 2,1 m³ wurden 1385 kg eines mit Talgfettalkohol gestarteten Ethoxylats mit durchschnittlich 25 Einheiten Ethylenoxid, 305 kg Methacrylsäuremethylester, 10,2 kg Titantetraisopropylat, 1,17 kg Butylhydroxyanisol, 850 g N,N-Diethylhydroxylamin und 277 g 2-Methyloxazolin vorgelegt. Es wurde unter Durchleiten von Stickstoff auf 120°C erwärmt und begonnen das gebildete Methanol zusammen mit überschüssigem Methacrylsäuremethylester abzudestillieren. Anschließend wurde die Temperatur innerhalb von 9 Stunden langsam auf 150°C erhöht und dort für 2 Stunden gehalten. Über NMR wurde ein Umsetzungsgrad von 94 % ermittelt. Nach dem Entleeren konnten im Kessel und in den Anlagenteilen zur Destillation keine Ablagerungen oder Verschmutzungen durch Polymerisate festgestellt werden.

## Patentansprüche

1. Verfahren zur Herstellung von Estern aus Alkoholen und olefinisch ungesättigten Carbonsäuren durch Umsetzung eines Alkohols mit einer olefinisch ungesättigten Carbonsäure oder einem reaktiven Derivat einer solchen, wobei 1 ppm bis 1 Gew.-%, bezogen auf das Gewicht des Reaktionsgemischs aus Alkohol und olefinisch ungesättigter Carbonsäure/Carbonsäurederivat, mindestens eines Oxazolins der Formel 1 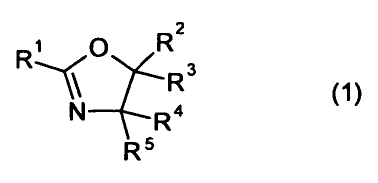 zugegen ist, worin R¹, R², R³, R⁴ und R⁵ unabhängig voneinander für Wasserstoff oder Kohlenwasserstoffreste mit 1 bis 12 Kohlenstoffatomen stehen, und R¹, R², R³, R⁴ und R⁵ gleich oder verschieden sein können.

2. Verfahren nach Anspruch 1, worin R¹, R², R³, R⁴ und R⁵ unabhängig voneinander für Wasserstoff oder Methylgruppen stehen.

3. Verfahren nach Anspruch 1 und/oder 2, worin
R¹ Methyl
R² und R³ Wasserstoff
R⁴ und R⁵ Wasserstoff oder Methyl bedeuten.

4. Verfahren nach einem oder mehreren der Ansprüche 1 bis 3, worin die Oxazoline der Formel 1 in Mengen von 10 ppm bis 0,5 Gew.-% bezogen auf das Reaktionsgemisch aus Alkohol und Carbonsäure/Carbonsäurederivat eingesetzt werden.

5. Verwendung von Verbindungen der Formel 1 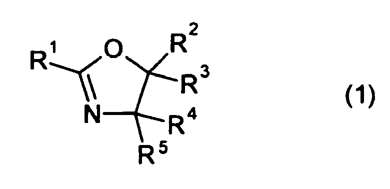 worin R¹, R², R³, R⁴ und R⁵ worin R¹, R², R³, R⁴ und R⁵ unabhängig voneinander für Wasserstoff oder Kohlenwasserstoffreste mit 1 bis 12 Kohlenstoffatomen stehen, und R¹, R², R³, R⁴ und R⁵ gleich oder verschieden sein können, als Stabilisatoren in der Umsetzung zwischen Alkoholen und olefinisch ungesättigten Carbonsäuren oder deren reaktiven Derivaten, wobei 1 ppm bis 1 Gew.-%, bezogen auf das Gewicht des Reaktionsgemischs aus Alkohol und Carbonsäure/Carbonsäurederivat, der Verbindung der Formel 1 zur Anwendung kommen.

6. Zusammensetzung, enthaltend
A) einen Alkohol
B) eine olefinisch ungesättigte Carbonsäure oder ein reaktives Derivat einer solchen,
wobei das molare Verhältnis zwischen A) : B) zwischen 1 : 0,2 und 1 : 15 liegt, sowie
C) 1 ppm bei 1 Gew.-% , bezogen auf das Gesamtgewicht von A) und B), einer Verbindung der Formel 1 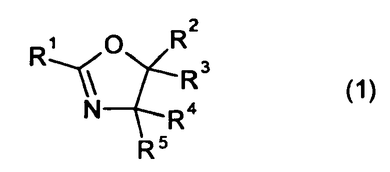
worin R¹, R², R³, R⁴ und R⁵ unabhängig voneinander für Wasserstoff oder Kohlenwasserstoffreste mit 1 bis 12 Kohlenstoffatomen stehen, und R¹, R², R³, R⁴ und R⁵ gleich oder verschieden sein können.

## Claims

1. A method for the preparation of esters from alcohols and olefinically unsaturated carboxylic acids by reacting an alcohol with an olefinically unsaturated carboxylic acid or a reactive derivative thereof, from 1 ppm to 1% by weight, based on the weight of the reaction mixture comprising alcohol and olefinically unsaturated carboxylic acid/carboxylic acid derivative, of at least one oxazoline of the formula 1 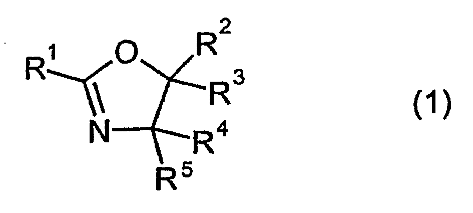 in which R¹, R², R³, R⁴ and R⁵ are independently hydrogen or hydrocarbon radicals having 1 to 12 carbon atoms which may be substituted by heteroatoms, and R¹, R², R³, R⁴ and R⁵ may be identical or different, being present.

2. The method as claimed in claim 1, wherein R¹, R², R³, R⁴ and R⁵, independently of one another, are hydrogen or methyl groups.

3. The method as claimed in claim 1 and/or 2, wherein
R¹ is methyl
R² and R³ are hydrogen
R⁴ and R⁵ are hydrogen or methyl.

4. The method as claimed in one or more of claims 1 to 3, wherein the oxazolines of the formula 1 are used in amounts of from 10 ppm to 0.5% by weight based on the reaction mixture comprising alcohol and carboxylic acid/carboxylic acid derivative.

5. The use of compounds of the formula 1 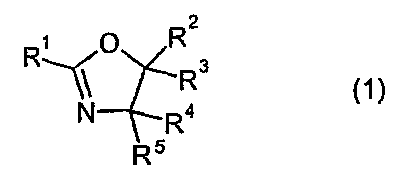 in which R¹, R², R³, R⁴ and R⁵ are independently hydrogen or hydrocarbon radicals having 1 to 12 carbon atoms, and R¹, R², R³, R⁴ and R⁵ may be identical or different, as stabilizers in the reaction between alcohols and olefinically unsaturated carboxylic acids or the reactive derivatives thereof, from 1 ppm to 1% by weight, based on the weight of the reaction mixture comprising alcohol and carboxylic acid/carboxylic acid derivative, of the compound of the formula 1 being used.

6. A composition comprising
A) an alcohol
B) an olefinically unsaturated carboxylic acid or a reactive derivative thereof,
the molar ratio A) : B) being from 1 : 0.2 to 1 : 15,
and
C) 1 ppm at 1% by weight, based on the total weight of A) and B), of a compound of the formula 1 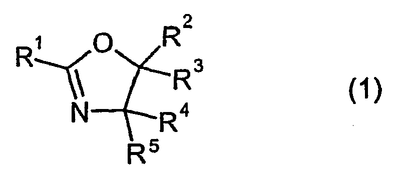
in which R¹, R², R³, R⁴ and R⁵ are independently hydrogen or hydrocarbon radicals having 1 to 12 carbon atoms, and R¹, R², R³, R⁴ and R⁵ may be identical or different.

## Revendications

1. Procédé pour la préparation d'esters d'alcools et d'acides carboxyliques oléfiniquement insaturés par transformation d'un alcool avec un acide carboxylique oléfiniquement insaturé ou un dérivé réactif de celui-ci, en présence de 1 ppm à 1% en poids, par rapport au poids du mélange réactionnel de l'alcool et de l'acide carboxylique/du dérivé de l'acide carboxylique oléfiniquement insaturé, d'au moins une oxazoline de formule 1 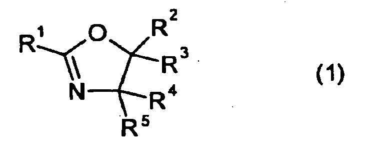 où R¹, R², R³, R⁴ et R⁵, indépendamment l'un de l'autre, représentent hydrogène ou des radicaux hydrocarbonés comprenant 1 à 12 atomes de carbone, et R¹, R², R³, R⁴ et R⁵ peuvent être identiques ou différents.

2. Procédé selon la revendication 1, dans lequel R¹, R², R³, R⁴ et R⁵, indépendamment l'un de l'autre, représentent hydrogène ou des groupes méthyle.

3. Procédé selon la revendication 1 et/ou 2, dans lequel
R¹ signifie méthyle
R² et R³ signifient hydrogène
R⁴ et R⁵ signifient hydrogène ou méthyle.

4. Procédé selon l'une ou plusieurs des revendications 1 à 3, dans lequel les oxazolines de formule 1 sont utilisées en des quantités de 10 ppm à 0,5% en poids par rapport au mélange réactionnel d'alcool et d'acide carboxylique/dérivé d'acide carboxylique.

5. Utilisation de composés de formule 1 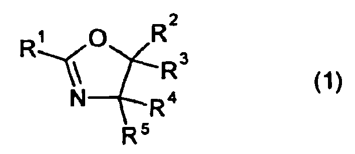 dans laquelle R¹, R², R³, R⁴ et R⁵, indépendamment l'un de l'autre, représentent hydrogène ou des radicaux hydrocarbonés comprenant 1 à 12 atomes de carbone, et R¹, R², R³, R⁴ et R⁵ peuvent être identiques ou différents, comme stabilisateurs dans la transformation entre des alcools et des acides carboxyliques oléfiniquement insaturés ou leurs dérivés réactifs, avec utilisation de 1 ppm à 1% en poids, par rapport au poids du mélange réactionnel de l'alcool et de l'acide carboxylique/du dérivé d'acide carboxylique, du composé de formule 1.

6. Composition contenant
A) un alcool
B) un acide carboxylique oléfiniquement insaturé ou un dérivé réactif de celui-ci,
le rapport molaire entre A):B) étant situé entre 1:0,2 et 1:15, ainsi que
C) 1 ppm à 1% en poids, par rapport au poids total de A) et B), d'un composé de formule 1 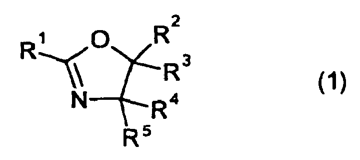
où R¹, R², R³, R⁴ et R⁵, indépendamment l'un de l'autre, représentent hydrogène ou des radicaux hydrocarbonés comprenant 1 à 12 atomes de carbone, et R¹, R², R³, R⁴ et R⁵ peuvent être identiques ou différents.
